# EUROPEAN PATENT APPLICATION

(11) **EP 1 260 255 A1**
(43) Date of publication of application: **27.11.2002**
(21) Application number: 01901609.6
(22) Date of filing: 10.01.2001
(51) Int. Cl.: A63J 23/02, A61J 17/00, A61L 9/00

(54) **METHOD FOR MUSIC RECORDING PLAYBACK WITH SIMULTANEOUS CO-ORDINATED AROMATIZATION OF THE ENVIRONMENT AND DEVICES FOR THE IMPLEMENTATION THEREOF**

(30) Priority: 10.01.2000 RU 2000100204
(71) Applicant: Tatevosyan, Ervand Iosifovich, Moscow, 121552 (RU)
(72) Inventor: Tatevosyan, Ervand Iosifovich, Moscow, 121552 (RU)
(74) Representative: Zellentin, Rüdiger, Dr.
(86) International application number: RU0100008
(87) International publication number: WO01051158

(57) **Abstract**

The invention relates to a technique for aromatizing air and can be used in every day life, during games and in medicine for creating systems for emotional and psychological relief with the use of music. Aromatizing and neutralising substances are coded by means of preliminary determined idientification codes. Coding signals for controlling the aromatization at the air include the sequence of identification codes of said substances which are to be introduced in the environment simultaneously and in harmony with the music playback recorded on a record carrier. The inventive method ensures a higher expressiveness of music playback, gives the music new qualitative nuances increases the pleasure of listening music due to integrated harmonious action on the sense organs of human beings.

## Description

### Field of the Invention

The present invention relates to air flavoring methods and is applicable in household and games, as well as in medicine for creating emotional and psychological relaxation systems using music, and may serve both entertainment and treatment and prophylaxis purposes.

### Description of the Prior Art

Considering reproduction of a music record in coordination with flavoring of ambient air as a unified process of creation of a kind of "aroma-music", the present invention may be referred to as a method for creating "aroma-music" and an aroma-music apparatus.

A color music apparatus is known in the art, which comprises a rotor with a mirror surface, an illuminated upper reservoir and a reservoir with atomized liquid, the reservoirs being connected to one another by threads and transparent elastic pipes which are passed through and secured on the rotor and through which the liquid is pumped when the rotor revolves; an atomizing nipple on the upper reservoir discharges the moisture with desired e.g. treating/recreating additives (see RF Patent No.1711942, IPC A63J 17/00, publ.15.02.91). The prior art apparatus, however, has elaborate design and restricted functional capabilities and consumer properties because it is impossible to change flavors, and the music record reproduction is uncoordinated with supply of the flavored stream.

Known in the art are apparatuses for discharging volatile substances to ambient air (see e.g. RF patents No.2043121, IPC A61L 9/03, publ.10.09.1995; No.2008924, IPC A61L 9/00, publ.30.05.1991). However, such conventional apparatuses and methods fail to provide reproduction of a music matter in coordination with flavoring the ambient air, which could improve expressiveness of the music matter reproduction and depth of its perception, as well as improve efficiency of treatment and recreation effect in aroma therapy procedures. Therefore, the apparatuses of such kind can not be treated as prior art with respect to the present invention.

A method is known for reproducing a music audio record simultaneously with coordinated flavoring of ambient air, comprising the steps of: recording air flavoring control signals on a record medium along with a music matter; reproducing from the record medium the recorded matter and the air flavoring control signals which are provided to an executive unit to act on appropriate reservoirs containing flavoring and neutralizing substances. The same prior art document describes an apparatus for reproducing a music audio record simultaneously with coordinated flavoring of ambient air, the apparatus comprising the following interrelated units: a record medium with a music matter and synchronously recorded air flavoring control signals; a sound reproducer for reproducing the recorded matter synchronously with said control signals; an ambient air flavoring device including a number of reservoirs with flavoring and neutralizing substances; an executive unit and a communication channel to transmit the air flavoring control signals from the sound reproducer to the executive unit (SU 391833, 19.12.1973).

### Summary of the Invention

The object of the present invention is to provide a method and apparatus which would improve efficiency of music matter perception by accompanying flavoring of ambient air, i.e. change of flavors in accordance with character and content of the music matter. This improves the reproduction expressiveness, depth of emotional influence on listeners owing to the created effect of simultaneous complex harmonic influence on human sense organs and qualitatively new nuances lend to listening music works.

The above object is attained in a method in accordance with the present invention comprising the steps of: pre-encoding flavoring and neutralizing substances with certain identification codes and generating encoded air flavoring control signals as sequences of identification codes of said substances which are to be discharged to ambient air simultaneously and in coordination with reproduction of a music matter; decoding said recorded encoded control signals to extract the flavoring and neutralizing substance identification codes, and selectively acting through the decoded control signals on the reservoirs with the substances in accordance with a sequence of said extracted flavoring and neutralizing substance identification codes.

The flavoring and neutralizing substances are preferably substances evaporating when heated, the appropriate flavoring and neutralizing substances being discharged by short time heating to a temperature of intensive evaporation of content of said reservoirs.

The encoded air flavoring control signals are preferably transmitted to an executive unit via a cable from a linear output of a sound reproducer used for recording.

The encoded air flavoring control signals are preferably transmitted to the executive unit by wireless method as acoustic vibrations.

The above object is attained in an apparatus in accordance with the invention, wherein an executive unit comprises a receiver for receiving encoded air flavoring control signals and a decoding unit for extracting the identification codes of appropriate flavoring and neutralizing substances, the decoding unit having an input connected to an output of said receiver, and an output connected to a control input of an ambient air flavoring device to selectively act on reservoirs with flavoring and neutralizing substances.

A communication channel for transmitting the encoded flavoring control signals is preferably a cable connecting a linear output of a sound reproducer to an input of the executive unit.

The communication channel for transmitting encoded flavoring control signals is preferably an acoustic vibration transmission channel, the executive unit comprising sensors to receive said control signals and a decoding unit connected to the sensors, said decoding unit having an output connected to a control input of the ambient air flavoring device.

The flavoring and neutralizing substances are preferably substances evaporating when heated, the apparatus further comprising means for short time heating up to a temperature of intensive evaporation of content of said reservoirs, said short time heating means being actuated by command from the executive unit.

The ambient air flavoring apparatus comprises a base for mounting reservoirs with flavoring and neutralizing substances in the form of removable and replaceable aerosol containers, and a number of magnetic driving members controlled by the decoded control signals to act on the aerosol containers.

A magnetic driving member preferably includes an electric magnet and a constant magnet moved by the electric magnet and applying pressure to an atomizing head of the aerosol container through an annular member fixed to the constant magnet, said annular member arranged coaxially with the atomizing head of the aerosol container and acting on the atomizing head when the constant magnet moves. Positions for mounting the aerosol containers on the base are enumerated, each of the positions corresponding to a container with a certain flavoring substance.

It should be taken into consideration that flavoring substances in this description refer to liquid flavors, natural volatile oils, extracts and compositions thereof, as well as similar odorous substances used in perfumery and cosmetics, food and pharmaceutics industry for household purposes and for treatment and recreation.

### Brief Description of the Drawings

The present invention will become apparent to those skilled in the arc from the following description of its preferred embodiments with reference to the accompanying drawings, in which:
Fig.1 shows a schematic simplified view of an apparatus for reproducing a music audio record simultaneously with coordinated flavoring of ambient air;
Fig.2 is a fragmentary view of an ambient air flavoring device;
Figs 3a, b, c are embodiments of an ambient air flavoring device;
Figs 4a, b, c are several embodiments of an apparatus of Fig.1 in the form of personal portable miniature apparatuses.

### Detailed Description of the Embodiments

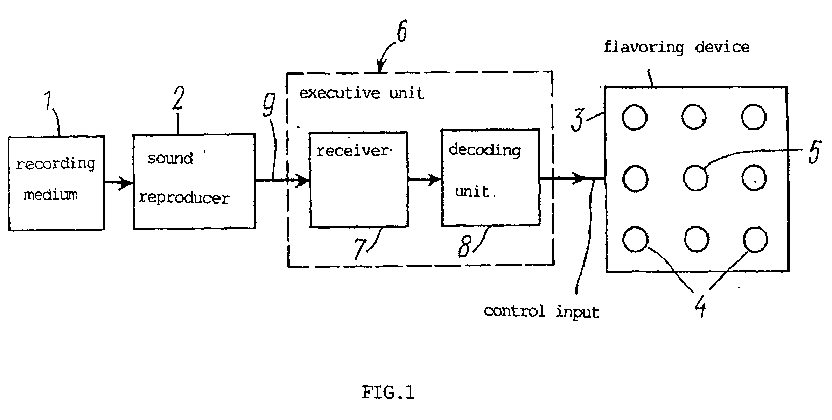

Referring now to Fig.1, an apparatus for reproducing a music audio record simultaneously with coordinated flavoring of ambient air comprises a record medium 1 with recorded music matter and encoded ambient air flavoring control signals, a sound reproducer 2 for reproducing the music matter simultaneously with the encoded control signals, an ambient air flavoring device 3 including a number of reservoirs 4 with flavoring substances and a reservoir 5 with a neutralizing substance, an executive unit 6 including a receiver 7 for receiving encoded flavoring control signals and a decoding unit 8 whose output is connected to a control input of the ambient air flavoring device 3. A communication channel for transmitting an encoded air flavoring control signal from the sound reproducer 2 to the executive unit 6 may be a cable 9 connecting a linear output of the sound reproducer 2 to the executive unit 6. In case of wireless transmission of the encoded control signal as acoustic vibrations, the operation radius will depend on the volume of the reproduced record, and a transmitting radio equipment may be used to transmit the encoded control signals at even greater distances between the sound reproducer and the executive unit.

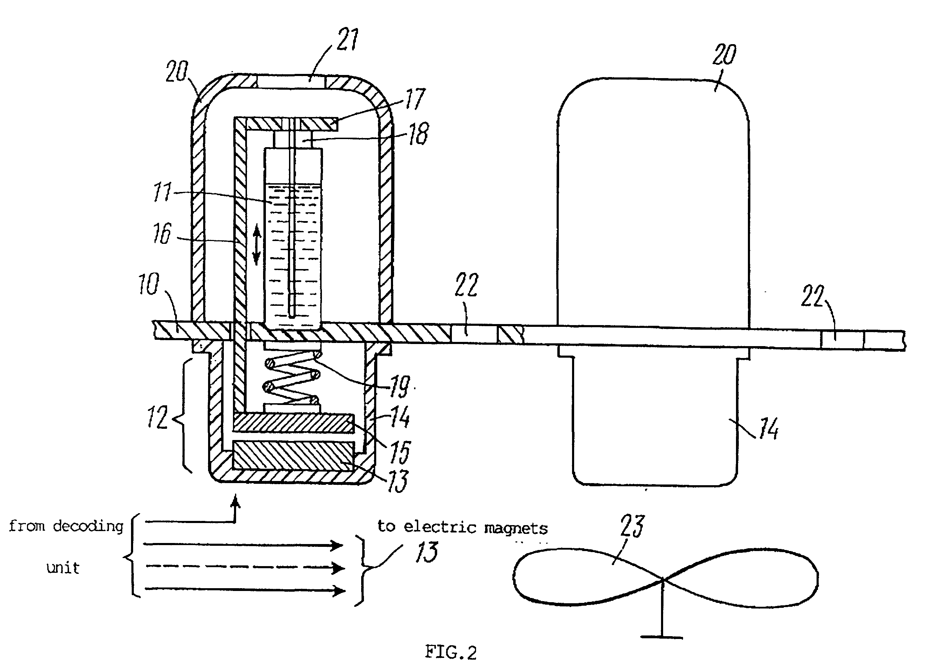

The ambient air flavoring device 3 comprises a base 10 for mounting reservoirs with flavoring and neutralizing substances, respectively, formed as removable and replaceable cylindrical aerosol containers 11 (see Fig.2) equipped with magnetic driving members 12 controlled by the decoded control signal to act on the aerosol containers 11. The magnetic driving member 12 may comprise, as shown in Fig.2, an electric magnet 13 fixed to the base 10 by a retainer (such as a bracket) 14 and a constant magnet in the form of a disk 15 fixed via a post 16 to an annular member 17 arranged coaxially with an atomizing head 18 of the aerosol container 11. A spiral spring 19 is secured by one end at the center of the disk 15 and by the one end to the base 10 under the aerosol container 11. From above the aerosol containers 11 may be closed by caps 20 with outlet orifices 21, the caps being attached to the base 10 by any suitable means, e.g. by thread. The base is also provided with through holes 22 to supply a controlled air flow from a built-in electric fan 23. The apparatus operates from electric mains or rechargeable batteries (not shown).

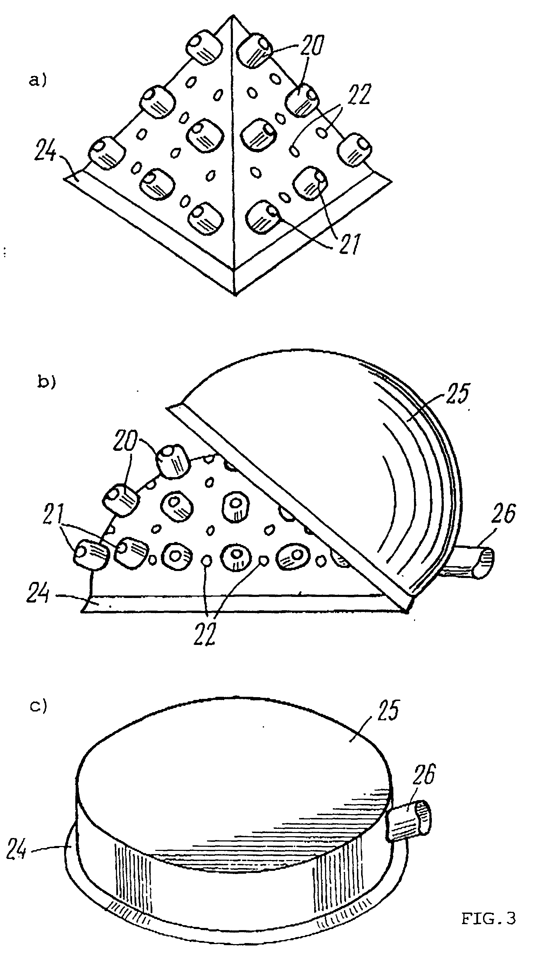

As seen in Figs 3a, b, c, the ambient air flavoring device 3 may be implemented in various shapes, e.g. cylinder, semi-sphere, pyramid, disk, etc., mounted on a support 24 and closed by a decorative cap 25 of appropriate shape with an outlet neck 26.

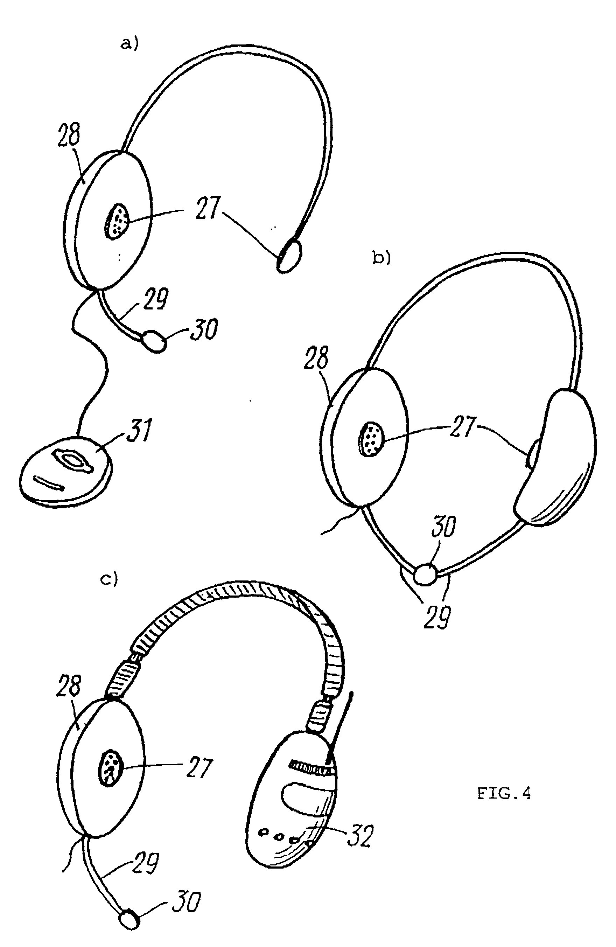

The ambient air flavoring device may be a personal portable miniature device, such as headphones or a telephone handset accommodating an executive unit and a replaceable set of minicontainers with flavoring and neutralizing substances, wherein the sound reproducer comprises headphones integrated in the flavoring device housing, in particular multimedia headphones compatible in operation with any audio- video- and telecommunication systems, including portable TV and radio apparatuses, audio and video CD-players, pocket-size computers etc., this significantly extending capabilities of their application. By way of example, the use of a pocket-size computer will enable editing the encoded flavoring control signals so that to adjust the aroma music creation process to individual taste. Examples of such apparatuses are illustrated in Figs 4a, b, c.

Figs 4a, b show an apparatus comprising headphones 27 as a sound reproducer integrated in a flavoring device 28 from which flavoring and neutralizing substances are to be blown off by a miniature fan (not shown) to an outlet pipe (pipes) 29 provided with an end cap 30 and supplied to user's olfactory organs. A music record will be transmitted to the headphones 27 from a sound reproducing apparatus 31. This structure may be implemented for one-side (Fig.4a) and two-side (Fig.4b) supply of flavoring substance.

Fig.4c shows an apparatus comprising the same components 27 to 30, but in this case a music record will be transmitted to the headphones 27 from a miniature music center 32 also mounted on the structure housing. Such a structure apparently permits combining the flavoring device with miniature sound reproducers.

An apparatus for reproducing music record simultaneously with coordinated flavoring of ambient air operates in the following fashion.

Flavoring and neutralizing substances are pre-encoded by certain identification codes. Along with a music matter at a record medium recorded are encoded flavoring control signals including sequences of flavoring substance identification codes corresponding to character and content of the recorded music matter, which must be supplied simultaneously and in coordination with reproduction of the music matter. The music matter and the encoded flavoring control signals are reproduced from the record medium 1 by a sound reproducer 2, and the encoded signals are provided over a communication channel 9 (e.g. a cable) via a receiver 7 to a decoding unit 8 of an executive unit 6. A decoding unit 8 decodes the received control signals and extracts identification codes of appropriate flavoring substances. The signals produced by the decoding unit are provided to a control input of a flavoring device 3, so that to selectively act, simultaneously with music matter reproduction, on reservoirs 4 with flavoring substances and, if appropriate, on a reservoir 5 with a neutralizing substance to neutralize the created aroma before acting on a next reservoir 4 with a flavoring substance.

The decoded signal provided to the ambient air flavoring device 3 from the executive unit 6 is fed to a magnetic driving member 12 (Fig.2), this resulting in current flow through an electromagnet 13 corresponding to the aerosol container 11 identified by the decoded identification code. The electromagnet 13 attracts a constant magnet 15 which by moving and stretching a spring 19 displaces an annular member 17 acting on an atomizing head 18 of the aerosol container 11 to provide a dosed output of the container content through an outlet opening 21. As the control signal ends, the current flow though the electric magnet 13 terminates and the disk 15 returns to its initial position under the action of the compressing spring 19. Upon discharge of the flavoring substance and before creation of the next flavor identified by the next identification code, the acting flavor may be neutralized, this being effected similarly to the described above procedure with only difference that the decoded control signal acts on the magnet driving member 12 corresponding to the aerosol container 11 with a neutralizing substance. The apparatus is then ready to a new cycle of next flavor creation initiated by decoding the next identification code in the control signal.

Intensity, i.e. dose of the flavoring substance supply depends on the record duration of the encoded signal corresponding to a particular identification code. It should be noted that when several encoded signals of the same or different duration pass in parallel in the record, flavoring substances of several kinds are simultaneously discharged, this resulting in different combinations obtained.

In one of embodiments, an apparatus in accordance with the present invention can operate autonomously without association with a sound reproducer and be controlled by an infra-red remote control unit. A control signal is generated in this embodiment with the aid of enumerated buttons as certain combinations of digits (numbers of buttons) corresponding to particular flavoring substances.

### Industrial Applicability

In future it would be advantageous to unify identification codes of flavoring and neutralizing substances in a unified international standard system and this will extend the application capabilities of the method in accordance with the invention even more. By way of example, connection to viewing or listening not only regional, but international music TV and radio programs as well, using sound record media with synchronous record of encoded ambient air flavoring control signals, including, e.g. connection to telephone, computer networks, will accordingly provide simultaneous effects of ambient air flavoring for even greater number of users.

## Claims

1. A method for reproducing a music audio record simultaneously with coordinated flavoring of ambient air, comprising
recording air flavoring control signals at a record medium with a music matter, reproducing from the record medium the recorded music matter and the air flavoring control signals which are provided to an executive unit to act on appropriate reservoirs containing flavoring and neutralizing substances,
said method **characterised by**:
pre-encoding flavoring and neutralizing substances by certain identification codes and generating encoded air flavoring control signals as sequences of identification codes of said substances which are be discharged to ambient air simultaneously and in coordination with reproduction of the music matter;
decoding said recorded encoded control signals to extract the flavoring and neutralizing substance identification codes and selectively acting by the decoded control signals on said reservoirs with said substances in accordance with a sequence of the extracted flavoring and neutralizing substance identification codes.

2. The method as set forth in claim 1, wherein said flavoring and neutralizing substances are substances evaporating when heated, the appropriate flavoring and neutralizing substances being discharged by short time heating to a temperature of intensive evaporation of content of said reservoirs.

3. The method as set forth in claim 1 or 2, wherein said encoded air flavoring control signals are transmitted to the executive unit via a cable from a linear output of a sound reproducer used for recording.

4. The method as set forth in claim 1 or 2, wherein said encoded air flavoring control signals are transmitted to the executive unit by wireless method as acoustic vibrations.

5. An apparatus for reproducing a music audio record simultaneously with coordinated flavoring of ambient air, comprising the interrelated units: a record medium with a music matter and synchronously recorded air flavoring control signals, a sound reproducer for reproducing the recorded music matter synchronously with said control signals, an ambient air flavoring device including a number of reservoirs with flavoring and neutralizing substances, an executive unit and a communication channel to transmit the air flavoring control signals from the sound reproducer to the executive unit,
said apparatus **characterized in that**
said executive unit comprises a receiver for receiving the air flavoring encoded control signals and a decoding unit for extracting the identification codes of appropriate flavoring and neutralizing substances, the decoding unit having an input connected to an output of said receiver, and an output connected to a control input of the ambient air flavoring device to selectively act on the reservoirs with flavoring and neutralizing substances.

6. The apparatus as set forth in claim 5, wherein said communication channel for transmitting encoded flavoring control signals is a cable connecting a linear output of the sound reproducer to an input of the executive unit.

7. The apparatus as set forth in claim 5, wherein said communication channel for transmitting encoded flavoring control signals is an acoustic vibration transmission channel, the executive unit including sensors to receive said control signals and a decoding unit connected to the sensors, said decoding unit having an output connected to a control input of the ambient air flavoring device.

8. The apparatus as set forth in any one of claims 5 to 7, wherein said flavoring and neutralizing substances are substances evaporating when heated, the apparatus further comprising means for short time heating up to a temperature of intensive evaporation of content of said reservoirs, said heating means being actuated by command from the executive unit.

9. The apparatus as set forth in claim 5, wherein said ambient air flavoring device comprises a base for mounting the reservoirs with flavoring and neutralizing substances formed as removable and replaceable aerosol containers, and a number of magnetic driving members controlled by the decoded control signals to act on said aerosol containers.

10. The apparatus as set forth in claim 9, wherein said magnetic driving member includes an electric magnet and a constant magnet moved by the electric magnet and applying pressure to an atomizing head of the aerosol container through an annular member fixed to the constant magnet, said annular member arranged coaxially with the atomizing head of the aerosol container and acting on the atomizing head when the constant magnet moves.

## Amended claims

### Amended claims under Art. 19.1 PCT

**1.** A method for reproducing a music audio record simultaneously with coordinated flavoring of ambient air, comprising
recording air flavoring control signals at a record medium with a music matter, reproducing from the record medium the recorded music matter and the air flavoring control signals which are provided to an executive unit to act on appropriate reservoirs containing flavoring and neutralizing substances,
said method **characterised by**:
pre-encoding flavoring and neutralizing substances by certain identification codes and generating encoded air flavoring control signals as sequences of identification codes of said substances which are be discharged to ambient air simultaneously and in coordination with reproduction of the music matter;
decoding said recorded encoded control signals to extract the flavoring and neutralizing substance identification codes and selectively acting by the decoded control signals on said reservoirs with said substances in accordance with a sequence of the extracted flavoring and neutralizing substance identification codes.

**2.** The method as set forth in claim 1, wherein said flavoring and neutralizing substances are substances evaporating when heated, the appropriate flavoring and neutralizing substances being discharged by short time heating to a temperature of intensive evaporation of content of said reservoirs.

**3.** The method as set forth in claim 1 or 2, wherein said encoded air flavoring control signals are transmitted to the executive unit via a cable from a linear output of a sound reproducer used for recording.

**4.** The method as set forth in claim 1 or 2, wherein said encoded air flavoring control signals are transmitted to the executive unit by wireless method as acoustic vibrations.

**5.** An apparatus for reproducing a music audio record simultaneously with coordinated flavoring of ambient air, comprising the interrelated units: a record medium with a music matter and synchronously recorded air flavoring control signals, a sound reproducer for reproducing the recorded music matter synchronously with said control signals, an ambient air flavoring device including a number of reservoirs with flavoring and neutralizing substances, an executive unit and a communication channel to transmit the air flavoring control signals from the sound reproducer to the executive unit,
said apparatus **characterized in that**
said executive unit comprises a receiver for receiving the air flavoring encoded control signals and a decoding unit for extracting the identification codes of appropriate flavoring and neutralizing substances, the decoding unit having an input connected to an output of said receiver, and an output connected to a control input of the ambient air flavoring device to selectively act on the reservoirs with flavoring and neutralizing substances.

**6.** The apparatus as set forth in claim 5, wherein said communication channel for transmitting encoded flavoring control signals is a cable connecting a linear output of the sound reproducer to an input of the executive unit.

**7.** The apparatus as set forth in claim 5, wherein said communication channel for transmitting encoded flavoring control signals is an acoustic vibration transmission channel, the executive unit including sensors to receive said control signals and a decoding unit connected to the sensors, said decoding unit having an output connected to a control input of the ambient air flavoring device.

**8.** The apparatus as set forth in any one of claims 5 to 7, wherein said flavoring and neutralizing substances are substances evaporating when heated, the apparatus further comprising means for short time heating up to a temperature of intensive evaporation of content of said reservoirs, said heating means being actuated by command from the executive unit.

**9.** The apparatus as set forth in claim 5, wherein said ambient air flavoring device comprises a base for mounting the reservoirs with flavoring and neutralizing substances formed as removable and replaceable aerosol containers, and a number of magnetic driving members controlled by the decoded control signals to act on said aerosol containers.

**10.** The apparatus as set forth in claim 9, wherein said magnetic driving member includes an electric magnet and a constant magnet moved by the electric magnet and applying pressure to an atomizing head of the aerosol container through an annular member fixed to the constant magnet, said annular member arranged coaxially with the atomizing head of the aerosol container and acting on the atomizing head when the constant magnet moves.

**11.** The apparatus as set forth in any one of claims 5 to 10, wherein said ambient air flavoring device is a personal portable device, such as headphones, with an integrated executive unit and a sound reproducer in the form of headphones, in particular multimedia headphones compatible in operation with various audio-, video-, telecommunication systems, including portable ones.
